**Europäisches Patentamt**

**European Patent Office**

**Office européen des brevets**

(19)

(11) Publication number : **0 346 472 B1**

(12) # EUROPEAN PATENT SPECIFICATION

(45) Date of publication of patent specification :
**15.07.92 Bulletin 92/29**

(51) Int. Cl.⁵ : **A61K 9/10,** A61K 35/18

(21) Application number : **88902212.5**

(22) Date of filing : **26.02.88**

(86) International application number :
**PCT/JP88/00207**

(87) International publication number :
**WO 88/06436 07.09.88 Gazette 88/20**

(54) PROCESS FOR PREPARING LIPOSOMES.

(30) Priority : **27.02.87 JP 42825/87**

(43) Date of publication of application :
**20.12.89 Bulletin 89/51**

(45) Publication of the grant of the patent :
**15.07.92 Bulletin 92/29**

(84) Designated Contracting States :
**BE CH DE FR GB IT LI SE**

(56) References cited :
**JP-A- 607 934
JP-A-59 134 712
JP-A-61 509 12
US-A- 4 133 874
No further relevant documents have been dis-
closed.**

(73) Proprietor : **TERUMO KABUSHIKI KAISHA
No. 44-1, Hatagaya 2-chome, Shibuya-ku
Tokyo 151 (JP)**

(72) Inventor : **SAKAGUCHI, Keisuke
Terumo Kabushiki Kaisha 2656-1, Ohbuchi
Fuji-shi Shizuoka 417 (JP)**

(74) Representative : **Gillard, Marie-Louise et al
Cabinet Beau de Loménie 55, Rue
d'Amsterdam
F-75008 Paris (FR)**

## Description

### Technical Field

The present invention relates to an improved method for preparing liposomes. More particularly, it is concerned with a method for preparing liposomes by hydrating liposome membrane-constituting materials at a temperature higher than the phase transition temperature (Tc), then adding an aqueous solution art or below the Tc followed by stirring.

Liposomes are widely used in the field of medicine as a carrier for a variety of physiologically active substances.

The method of the invention is particularly favorable for the production of the liposomes into which a substance being unstable at higher temperatures is to be incorporated.

### Background Technology

Liposomes are prepared by dissolving liposome membrane-constituting materials in an appropriate organic solvent such as chloroform, distilling off the solvent from the resulting solution to form a lipid film to which is then added an aqueous solution of a drug followed by vigorously stirring to an aqueous suspension and then treating the suspension with such a sonication. It is known that the preparation of liposomes is necessary to be carried out at a temperature not lower than the phase transition temperature (gel-liquid crystal phase transition temperature, Tc) and that at a temperature lower than Tc a homogeneous suspension will not be produced when the lipid is suspended in water (Saishin Igaku [Modern Medicine] Vol. 37, No. 2, pp. 338-345). Thus, when dipalmitoylphosphatidyl-choline (DPPC) which has a Tc of 41°C is employed as a membrane-constituting material, suspension must be made at a temperature not lower than 41°C so that it is very difficult to incorporate a substance extremely unstable at higher temperatures such as a biological substance (for example, hemoglobin) into the inner aqueous space.

A process for preparing freeze-dried liposomes which comprises dispersing multilamella or lunilamella vesicles in an aqueous medium containing active ingredients at or over a temperature of the gel-phase/liquid crystal-phase transition is disclosed in EP 0 171 710.

As a result of extensive studies we have found that liposome membrane-constituting materials including phospholipids and cholesterol when hydrated at a temperature not lower than the Tc form liposomes with an aqueous solution held even if liposome formation-, that is, incorporation of the aqueous solution into liposomes is carried out at a temperature not higher than the Tc.

When phospholipids are completely hydrated to form a lamella phase (lipid bilayers), liposome formation readily occurs. If, however, phospholipids are mixed with water at a temperature not higher than the Tc, water hardly enters the hydrophilic group due to the hydrocarbon chains of phospholipid regularly arranged, so that the hydration is difficult. Therefore, when phospholipids are mixed with water at a temperature not lower than the Tc, preferably not lower than the Tc in powdery crystal, it is conceived that the hydrocarbon chains of the phospholipids are in "melted" state, and the hydration is easy. It is also believed that when phospholipids hydrated to lamella phase are cooled to a temperature at or below the Tc, it will become gelled state, which makes formation of liposomes difficult, but when cholesterol added, the branched paraffin chain of the cholesterol will enter the central portion of lipid bilayers resulting in increases fluidity at the central portion, and liposomes may be formed readily from gel-like lipid thus formed. Thus, hydrated phospholipids containing cholesterol are capable of forming liposomes at a temperature not higher than the Tc so that substances being unstable at a higher temperature can be incorporated into the inner aqueous space.

The present invention has been completed on the basis of the above-described findings, the object of which is to provide a method for preparing liposomes allowing for production of liposomes at a temperature not higher than Tc of the liposome membrane-constituting materials.

### Disclosure of the Invention

The above-mentioned object is achieved by a method for preparing liposomes according to the invention as set forth below.

1) A method for preparing liposomes, which comprises the steps of :

    a) hydrating liposome membrane-constituting materials including phospholipids and cholesterol with water added; at a temperature higher than the phase-transition temperature (Tc) of the membrane-constituting materials, and

    b) adding an aqueous solution containing a physiologically active substance to the hydrated liposome membrane-constituting materials at a temperature art or below Tc and stirring the mixture to form liposomes.

2) A method according to the item 1 wherein the phospholipids are hydrogenated natural phospholipids or synthetic phospholipids composed of saturated fatty acids.

3) A method according to the item 2 wherein the hydrogenated natural phospholipids or the synthetic phospholipids composed of saturated fatty acids are hydrogenated natural lecithins or synthetic lecithins composed of saturated fatty acids.

4) A method according to any of the items 1 to 3 wherein the liposome membrane-constituting materials include a negative charge-providing substance.

5) A method according to the item 4 wherein the negative charge-providing substance is phosphatidic acid or dicetyl phosphate.

6) A method according to the item 1 wherein the physiologically active substance is hemoglobin.

The method of the invention is easily conducted by adding an appropriate amount of water to liposome membrane-constituting materials containing phospholipids and cholesterol, heating the mixture at a temperature higher than the phase transition temperature for hydration followed by addition of an aqueous solution and stirring the mixture.

There is no particular limitation on the nature of phospholipids in the invention, and any of those natural or synthetic phospholipids which form liposomes may be employed. In view of the object of the invention, however, those which have a relatively high Tc are used. Particularly preferable are hydrogenated natural lecithins or synthetic lecithins composed of saturated fatty acids. These phospholipids are hardly apt to form toxicant peroxides. As they are also stable to oxidation, they are especially favorable when a substance unstable to oxidation is to be incorporated into the inner aqueous space. The hydrogenated natural lecithins as mentioned above are hydrogenation products of the lecithins obtained from a natural material such as egg yolk or soybean. Degree of the hydrogenation is 30 or below, preferably 10 or below in terms of the iodine value. Cholesterol which is effective for increasing fluidity of the phospholipid membrane is preferably added in a ratio of 1 mole or more to 2 moles of the phospholipid. In addition to the phospholipids and the cholesterol, known additives such as a negative charge-providing substance for the prevention of agglutination (for example, phosphatidic acid or sodium salt thereof or dicetyl phosphate) and an antioxidant (for example, tocopherol) may be added as the liposome membrane-constituting material. These membrane-constituting materials are preferably employed at the ratios of 0.2-1.0 parts by weight of cholesterol and 0.1-0.3 parts by weight of a negative charge-providing substance to 1 part by weight of phospholipids.

There is no limitation on the aqueous solution to be incorporated into the inner aqueous space of liposomes, and aqueous solutions containing various physiologically active substances are used. In view of the features of the invention, however, physiologically active substances that are deteriorated or inactivated at higher temperatures such as hemoglobin, hormones, for example, insulin and hydrocortisone, anticancer agents and substances of living body origin such as enzyme preparations, for example, SOD (superoxide dismutase) are preferably used.

In carrying out the preparative method of the invention, first, membrane-constituting materials are completely dissolved in an organic solvent such as chloroform followed by removal of the solvent by such a means as reduced pressure or spraying of an inert gas to form a homogeneous mixture. Subsequently, distilled water is added, and the mixture is completely and wholly hydrated by such a procedure as kneading at a temperature higher than the phase transition temperature (Tc). Although the hydration is more easily effected with more amount of the distilled water, the aqueous solution of a drug will be diluted with the excess amount of water to result in reduction in holding the drug in liposomes. An adequate amount of the distilled water to be added is 1 milliliter per of the membrane-constituting materials. Then, the mass is maintained at such a temperature as causing no inactivation of a drug to be held and mixed with an aqueous solution of the drug followed by stirring. Further, the liquid mixture is dispersed by such a means as a pressure emulsifier, French pressure cell or sonicator while carefully observing the temperature. The operation is continued until an appropriate particle size is produced.

The liposomes prepared as above are subjected to such a procedure as dialysis, gel filtration, ultrafiltration or centrifugal separation to separate the free drug and may also be suspended in an aqueous solution for specific applications.

The invention will be described in more detail below with reference to an example and comparative examples.

Example

In 50 ml of chloroform were dissolved 2.90 g of hydrogenated egg yolk lecithin (Tc=45-60°C, $Tc_{max}$=55°C), 0.73 g of cholesterol and 0.39 g of phosphatidic acid sodium salt (from egg yolk lecithin), and the solution was placed in a 300-ml round-bottomed flask. The chloroform in the flask was removed by a rotary evaporator followed by vacuum drying to obtain a dried product of the membrane-constituting materials. To the product was added 4.0 ml of distilled water, and glass beads were put in the flask, which was shaken at 80°C (>Tc) for blending and hydration. The flask was then placed and cooled in a water bath maintained at 4°C (< Tc), to which was added 25 ml of 30% (w/v) hemoglobin solution. The mixture was well shaken to an almost homogeneous liquid, which was then subjected to 10 continuous treatments by passing through a French pressure cell (manufactured by Ohtake Seisakusho) at a pressure of $6867.10^4$Pa (700 kg/cm$^2$) at 4°C. After addition of 200 ml of physiological salt solution, the mixture was centrifuged (27000x g, for 30 min.) at 4°C. Precipitates thus obtained were suspended in 50 ml of physiological salt solution, and centrifugal separation and re-suspension were repeated until no color of hemoglobin was observed in the supernatant from centrifugation. The finally produced liposome suspension colored pale red and was observed in visible absorptiometry by a spectrophotometer (Model UV-300 manufactured by Shimazu) for a spectrum characteristic of oxyhemoglobin. Degradation by oxidation was not observed. A large number of spherical particles of approximately 1 μm or less in size were microscopically observed.

Ratio of the amount of hemoglobin held in the final liposome suspension to that added on preparation was 19.1% according to hemoglobin assay by the cyan methemoglobin method. The hemoglobin-containing liposomes thus obtained are useful as artificial erythrocytes.

Comparative Example 1

A dried product of the membrane-constituting materials was obtained in the same way as in the Example. To it was added 25 ml of 30% hemoglobin solution. The mixture was shaken at 65°C (>Tc). The solution gradually colored brown and formed precipitates which were considered to be degraded hemoglobin.

Comparative Example 2

A dried product of the membrane-constituting materials was obtained in the same way as in the Example. To it was added 25 ml of 30% hemoglobin solution. The mixture was shaken at 4°C (< Tc). The lipids were hardly hydrated and remained unchanged almost in solid. Subsequently, the mass was dispersed at 4°C by means of a bath type sonicator. Free hemoglobin was removed in the same manner as in the Example. The final liposome suspension colored milk white with pale red tint. Examination of the visible absorptiometry made in the same way as in the Example revealed that it had a slight absorption spectrum characteristic of hemoglobin. A number of distorted particles of several μm in size were microscopically observed. Ratio of the hemoglobin held in the final suspension as determined in the same way as in the Example was 2.3%.

Industrial Applicability

According to the invention, there is provided a method for preparing liposomes which comprises hydrating liposome membrane-constituting materials including phospholipids and cholesterol at a temperature higher than the phase transition temperature and then adding an aqueous solution at a temperature lower than the Tc followed by stirring.

In the present invention, whereas the hydration was carried out at a temperature higher than the Tc, the dispersion into aqueous solution was carried out at a temperature art or below the Tc so that the aqueous solution remains unheated even when membrane-constituting materials with a high Tc are employed and, therefore, aqueous solution containing a heat-unstable substance can be used.

According to the invention, there can be prepared liposomes which contain in the inner aqueous space a physiologically active substance liable to degradation or inactivation such as hemoglobin, hormones, e.g., insulin and hydrocortisone, anticancer drugs, or enzymatic preparations, e.g., SOD (superoxide dismutase).

Moreover, when hydrogenated natural phospholipids or synthetic phospholipids composed of saturated fatty acids are used as the phospholipids in the invention, not only such phospholipids have a high Tc and are very stable to oxidation of the membrane constituents, but also the inner aqueous space will hardly be influenced by the oxidation. Accordingly, the method is favorable for preparing liposomes containing a substance liable to oxidation.

## Claims

1. A method for preparing liposomes, which comprises the steps of :
   a) hydrating liposome membrane-constituting materials including phospholipids and cholesterol with water added, at a temperature higher than the phase-transition temperature (Tc) of the membrane-constituting materials, and
   b) adding an aqueous solution containing a physiologically active substance to the hydrated liposome membrane-constituting materials at a temperature art or below Tc and stirring the mixture to form liposomes.

2. A method according to Claim 1 wherein the phospholipids are hydrogenated natural phospholipids or synthetic phospholipids composed of saturated fatty acids.

3. A method according to Claim 2 wherein the hydrogenated natural phospholipids or the synthetic phospholipids composed of saturated fatty acids are hydrogenated natural lecithins or synthetic lecithins composed of saturated fatty acids.

4. A method according to any of Claims 1 to 3 wherein the liposome membrane-constituting materials include a negative charge-providing substance.

5. A method according to Claim 4 wherein the negative charge-providing substance is phosphatidic acid or dicetyl phosphate.

6. A method according to Claim 1 wherein the physiologically active substance is hemoglobin.

## Patentansprüche

1. Verfahren zur Herstellung von Liposomen, das die folgenden Schritte umfaßt:

a) Hydratisieren von Liposomenmembranen bildenden Stoffe, einschließlich Phospholipiden und Cholesterin, durch Zugabe von Wasser bei einer Temperatur, die über der Phasenübergangstemperatur (Tc) der membranbildenden Substanzen liegt, und

b) Zugabe einer wäßrigen, einen physiologisch aktiven Stoff enthaltenden Lösung zu den hydratisierten, die Liposomenmembran bildenden Stoffen bei einer Temperatur, die bei Tc oder darunter liegt, und Rühren des Gemisches zur Bildung von Liposomen.

2. Verfahren nach Anspruch 1, wobei die Phospholipide hydrierte natürliche Phospholipide oder aus gesättigten Fettsäuren aufgebaute synthetische Phospholipide sind.

3. Verfahren nach Anspruch 2, wobei die hydrierten natürlichen Phospholipide oder die aus gesättigten Fettsäuren bestehenden synthetischen Phospholipide hydrierte natürliche Lecithine oder aus gesättigten Fettsäuren aufgebaute synthetische Lecithine sind.

4. Verfahren nach einem der Ansprüche 1 bis 3, wobei die die Liposomenmembran bildenden Stoffe eine negative Ladung liefernde Substanz umfassen.

5. Verfahren nach Anspruch 4, wobei die negative Ladung liefernde Substanz Phosphatidsäure oder Dicetylphosphat ist.

6. Verfahren nach Anspruch 1, wobei der physiologisch aktive Stoff Hämoglobin ist.

## Revendications

1. Un procédé pour préparer des liposomes qui comprend les étapes suivantes :

a) on hydrate des substances constituant la membrane des liposomes contenant des phospholipides et du cholestérol par addition d'eau, à une température supérieure à la température de transition de phase (Tc) des substances constituant la membrane, et

b) on ajoute une solution aqueuse contenant une substance physiologiquement active aux substances constituant la membrane des liposomes hydratées à une température égale ou inférieure à Tc, et on agite le mélange pour former des liposomes.

2. Un procédé selon la revendication 1 dans lequel les phospholipides sont des phospholipides naturels hydrogénés ou des phospholipides synthétiques composés d'acides gras saturés.

3. Un procédé selon la revendication 2 dans lequel les phospholipides naturels hydrogénés ou les phospholipides synthétiques composés d'acides gras saturés sont des lécithines naturelles hydrogénées ou des lécithines synthétiques composées d'acides gras saturés.

4. Un procédé selon l'une quelconque des revendications 1 à 3 dans lequel les substances constituant la membrane des liposomes contiennent une substance fournissant une charge négative.

5. Un procédé selon la revendication 4 dans lequel la substance fournissant une charge négative est un acide phosphatidique ou le phosphate de dicétyle.

6. Un procédé selon la revendication 1 dans lequel la substance physiologiquement active est l'hémoglobine.